# EUROPÄISCHE PATENTSCHRIFT

(11) **EP 1 190 194 B1**
(45) Veröffentlichungstag und Bekanntmachung des Hinweises auf die Patenterteilung: **13.08.2003**
(21) Anmeldenummer: 00952973.6
(22) Anmeldetag: 28.06.2000
(51) Int. Cl.: F16K 15/14, A61M 39/24

(54) **ZWEI-WEGE-VENTIL, INSBESONDERE FÜR FLÜSSIGKEITSSPENDER**
TWO-WAY VALVE, ESPECIALLY FOR LIQUID DISPENSERS
SOUPAPE A DEUX VOIES, EN PARTICULIER POUR DISTRIBUTEUR DE LIQUIDE

(30) Priorität: 28.06.1999 DE 19929607
(43) Veröffentlichungstag der Anmeldung: 27.03.2002
(73) Patentinhaber: Ing. Erich Pfeiffer GmbH, 78315 Radolfzell (DE)
(72) Erfinder: MIJERS, J., W., M., NL-5913 TP Venlo (NL)
(74) Vertreter: Patentanwälte Ruff, Wilhelm, Beier, Dauster & Partner
(86) Internationale Anmeldenummer: EP0006023
(87) Internationale Veröffentlichungsnummer: WO01001023

(56) Entgegenhaltungen:
- DE-A- 19 816 398
- FR-A- 2 666 745
- US-A- 3 270 771
- US-A- 4 084 606

## Beschreibung

Die Erfindung betrifft ein Zwei-Wege-Ventil, insbesondere für Flüssigkeitsspender, mit einem ersten Anschluß für einen Flüssigkeitsvorratsbehälter, einem zweiten Anschluß für eine Pumpe zum Saugen oder Drücken und mit einem dritten Anschluß für einen Flüssigkeitsverbraucher, wobei eine Membranscheibe an ihrem radial äußeren Ringrand zwischen einem Ventilgehäuse und einem Deckelgehäuse eingespannt ist und flach gegen eine Ringsitzfläche anliegt, in deren Bereich sich eine axiale Öffnung für die Flüssigkeit befindet und wobei die Membranscheibe deckelseitig gegen eine Gegen-Ringsitzfläche anliegt.

Ein solches Zwei-Wege-Ventil ist aus der älteren, nicht vorveröffentlichten DE 198 16 398 A1 mit einem ersten Anschluß für einen Medikamentenvorratsbehälter, einem zweiten Anschluß für eine Pumpe (mit Kolben) zum Saugen oder Drücken und mit einem dritten Anschluß für einen Flüssigkeitsverbraucher bekannt.

Eine hierzu ähnliche Bauweise ergibt sich aus US 4,246,932.

Weiterhin ist es aus der DE 196 43 360 C1 bekannt, eine Membranscheibe mit vollem Querschnitt an ihrem radial äußeren Ringrand zwischen einem Ventilgehäuse und einem Deckelgehäuse einzuspannen und gegen eine Ringsitzfläche anliegen zu lassen. Im Bereich der Ringsitzfläche befindet sich eine axiale Öffnung für die Flüssigkeit, und die Membranscheibe liegt deckelseitig gegen eine andere Ringsitzfläche.

Derartige Zwei-Wege-Ventile werden in der Medizintechnik und in der Hygienetechnik, z.B. für Infusionsgeräte, Spraybehälter u. dgl., bzw. in Flüssigkeitsspendern für flüssige Seifen, Cremes, Suspensionen aller Art, Reinigungsmittel, Gels, Salben u. dgl. eingesetzt. Unter Flüssigkeit sind hier also alle Arten von Fluiden zu verstehen, insbesondere auch Suspensionen von Feststoffen in Fluiden sowie Gele, Schäume etc.

Die hierzu verwendeten Pumpsysteme bauen jeweils auf einer Einheit aus Anschlußstück, Dichtgehäuse, Kolben mit einer Druckfeder und mit einer Kugel auf. Da die Kugeln schon aus Gewichts- und Kostengründen aus Stahl oder Edelstahl gefertigt sind, finden zwischen der Flüssigkeit und dem Stahlwerkstoff unerwünschte chemisch-physikalische Reaktionen statt, so daß bei der Berührung einer Medikamentflüssigkeit mit der Kugel molekulare Prozesse auftreten, die das Medikament verändern und dadurch ein solches System verbieten.

Der Erfindung liegt die Aufgabe zugrunde, abweichend von dem bekannten Stahlkugel-Druckfeder-System und aufbauend auf Zwei-Wege-Ventilen mit Membranscheiben ein Zwei-Wege-Ventil mit gefedertem Kolben zu schaffen, durch den einzelne Portionen abgemessen und ausgegeben werden können.

Die gestellte Aufgabe wird beim eingangs bezeichneten zwei - Wege - Ventil erfindungsgemäß dadurch gelöst, daß zwischen dem radial äußeren Ringrand und der im Durchmesser radial kleineren Ringsitzfläche eine zusätzliche beidseitige Einspannung der im Zentrum offenen Membranscheibe, bestehend aus der Gegen-Ringsitzfläche und einer Ringsitzfläche, vorgesehen ist und daß neben der zusätzlichen Einspannung des ersten Anschlusses ein erster Ringkanal gebildet ist, der in den dritten Anschluß mündet. Dadurch wird Flüssigkeit bei einem Saughub des Kolbens angesaugt und durch einen nachfolgenden Druckhub durch den dritten Anschluß für den Flüssigkeitsverbraucher transportiert. Der Flüssigkeitstransport kann in abgestimmten Mengen je nach der Zähflüssigkeit der Flüssigkeit erfolgen.

Eine Ausgestaltung sieht vor, daß zwischen dem radial äußeren Ringrand der Membranscheibe und der zusätzlichen, radial inneren Einspannung die Membranscheibe gegen eine einseitige, ventilgehäuseseitige, ringförmige Auflage anliegt. Dadurch wird ein weiterer Ventilsitz geschaffen, so daß in dem Zwei-Wege-Ventil im Grunde genommen zwei Ventile vereinigt sind.

Nach weiteren Merkmalen ist vorgesehen, daß der ringförmigen Auflage am Ventilgehäuse ein Ringkanal des Deckelgehäuses gegenüberliegt. Der Vorteil besteht in der Schaffung eines volumenmäßig ausreichend großen Strömungskanals.

Eine Weiterentwicklung sieht vor, daß innerhalb des Ventilgehäuses, an die Auflage radial angrenzend, ein dem Ringkanal des Deckelgehäuses gegenüberliegender Anschlußkanal vorgesehen ist. Der Anschlußkanal sichert einen ausreichend großen Strömungsquerschnitt für die Abführung der Flüssigkeit im dritten Anschluß.

Für einen ausreichend großen Strömungsquerschnitt trägt sodann bei, daß auf der Seite des Ventilehäuses der ringförmige Anschlußkanal mit einer gegenüber der radialen Breite größeren axialen Länge vorgesehen ist. Hierbei entspricht die größere axiale Länge dem Durchmesser des Strömungskanals im dritten Anschluß.

Ferner sorgt für günstige Strömungsquerschnitte, daß der Ringkanal an einer Umfangstelle durch einen den Durchmesser der Membranscheibe umgebenden Kanalabschnitt im Deckelgehäuse und einem solchen Kanalabschnitt im Ventilgehäuse mit dem Anschlußkanal verbunden ist. Diese Maßnahme trägt auch dazu bei, einen unabhängigen Strömungskanal für den Druckvorgang des Kolbens zu schaffen.

Eine Verbesserung sieht ferner vor, daß in dem Deckelgehäuse die Ringsitzfläche mittels einer nach innen hohlen Ausnehmung eines Kernteils des Deckelgehäuses gebildet ist. Dadurch wird die erwähnte Ringsitzfläche für die Membranscheibe geschaffen.

Schließlich bestehen Erfindungsmerkmale darin, daß um den Kernteil des Deckelgehäuses einzelne ringsegmentförmige Ausnehmungen oder mehrere zylindrische Löcher angeordnet sind, die den ersten Anschluß mit dem Zentrum bei geöffnetem Ventil verbinden.

In der Zeichnung sind Ausführungsbeispiele der Erfindung dargestellt und werden nachfolgend näher erläutert.

Es zeigen:
- Fig. 1A: einen axialen Querschnitt durch das Zwei-Wege-Ventil in Saugstellung,
- Fig. 1B: einen axialen Querschnitt durch das Zwei-Wege-Ventil in Druckstellung,
- Fig. 2: eine Draufsicht auf das ohne Deckelgehäuse dargestellte Ventilgehäuse,
- Fig. 3: einen axialen Querschnitt in der Art nach Fig. 1 durch das Ventilgehäuse,
- Fig. 4: einen axialen Querschnitt durch das Deckelgehäuse und
- Fig. 5: eine Draufsicht auf das Deckelgehäuse gemäß Fig. 4.

Das Zwei-Wege-Ventil ist mit einem ersten Anschluß 1 für einen Flüssigkeitsvorratsbehälter 1a, einem zweiten Anschluß 2 für eine nicht näher dargestellte Pumpe versehen. Die Pumpe kann aus einem einfachen Kolben bestehen, der von Hand betätigt wird. Ferner ist ein dritter Anschluß 3 für einen Flüssigkeitsverbraucher vorgesehen. Als solcher kann ein Patient angesehen werden, dem eine medikamentöse Flüssigkeit verabreicht wird. Der dritte Anschluß 3 ist, wie gezeichnet, als Luer-Lock-Anschluß ausgeführt. Das Zwei-Wege-Ventil besitzt als wesentlichen Bestandteil eine Membranscheibe 4, die an ihrem radial äußeren Ringrand 4a zwischen einem Ventilgehäuse 5 und einem Deckelgehäuse 6 eingespannt ist, wobei das Ventilgehäuse 5 und das Deckelgehäuse 6 dauerhaft und dicht, z.B. durch Verschweißen von Kunststoffteilen, miteinander verbunden sind. Die Form der Anschlüsse 1, 2, 3 kann dabei frei gewählt werden. Bei medizinischen und medizintechnischen Anwendung können beispielsweise sogenannte Luer-Lock-Anschlüsse verwendet werden. Die Anschlüsse können dabei sowohl als männliche als auch als weibliche Anschlüsse ausgebildet sein. Die Membranscheibe 4 liegt gegen eine Ringsitzfläche 10 an, die durch eine besondere Formgestaltung zustande kommt. Im Bereich 7 der Ringsitzfläche 10 befindet sich eine axiale Öffnung 8 für die anzusaugende Flüssigkeit, die anschließend durch den dritten Anschluß 3 dem Flüssigkeitsverbraucher zugeführt wird. Die Membranscheibe 4 liegt deckelseitig gegen eine im Durchmesser gleichgroße Gegen-Ringsitzfläche 6a an.

Das derart gestaltete Zwei-Wege-Ventil bildet für den Saughub einen Durchfluß vom Flüssigkeitsvorratsbehälter 1a in einen Zylinderraum 21 und später für den Druckhub des Kolbens einen Durchfluß in den dritten Anschluß 3.

Diese Durchflüsse werden dadurch ermöglicht, daß zwischen dem radial äußeren Ringrand 4a der Membranscheibe 4 und einer im Durchmesser radial kleineren Ringsitzfläche 10 eine zusätzliche beidseitige Einspannung 9 der im Zentrum 4b offenen Membranscheibe 4 geschaffen ist, die aus der Gegen-Ringsitzfläche 6a und einer Ringsitzfläche 2a besteht. Neben der zusätzlichen Einspannung 9 im Hinblick auf die Einspannung am radial äußeren Ringrand 4a des ersten Anschlusses 1 ist ein erster Ringkanal 11 gebildet, der bei geöffnetem Ventil in den dritten Anschluß 3 mündet.

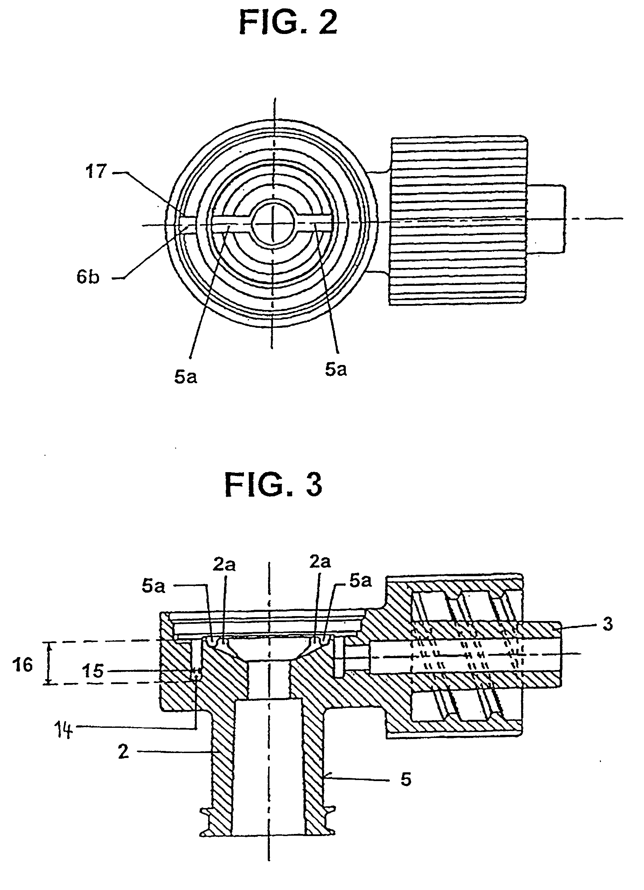

Zwischen dem radial äußeren Ringrand 4a der Membranscheibe 4 und der zusätzlichen, radial inneren Einspannung 9 liegt die Membranscheibe 4 gegen eine einseitige, ventilgehäuseseitige, ringförmige Auflage 12 an. Dabei liegt der ringförmigen Auflage 12 am Ventilgehäuse 5 ein zweiter Ringkanal 13 des Deckelgehäuses 6 gegenüber. Innerhalb des Ventilgehäuses 5 ist, an die Auflage 12 radial angrenzend, ein dem Ringkanal 13 des Deckelgehäuses 6 gegenüberliegender Anschlußkanal 14 vorhanden. Der ringförmige Anschlußkanal 14, der in dem Ventilgehäuse 5 durch Spritzen aus Kunststoff gebildet ist, besitzt eine gegenüber der radialen Breite 15 größere axiale Länge 16 (vgl. Fig. 3).

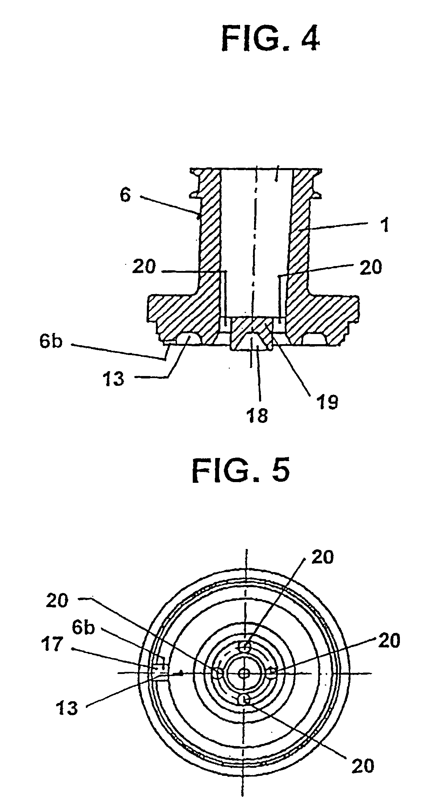

Der zweite Ringkanal 13 ist an einer Umfangstelle 17 mittels eines den Durchmesser der Membranscheibe 4 umgebenden Kanalabschnitts 6b (siehe auch Fig. 4) im Deckelgehäuse 6 und einem ergänzenden Kanalabschnitt 5a im Ventilgehäuse 5 mit dem Anschlußkanal 14 verbunden. In dem Ventilgehäuse 5 ist die Ringsitzfläche 10 mittels einer nach innen hohlen Ausnehmung 18 eines Kernteils 19 des Deckelgehäuses 6 geformt. Um den Kernteil 19 sind einzelne ringsegmentförmige Ausnehmungen oder (wie gezeichnet) mehrere zylindrische Löcher 20 angeordnet. Die Löcher 20 verbinden den Zylinderraum 21 für einen Saug-/Druckkolben im Bereich 7 mit dem ersten Ringkanal 11.

Dabei ist die Membranscheibe 4 gegenüber der Ringsitzfläche 10 und gegenüber der ringförmigen Auflage 12 vorgespannt. Die Vorspannung gegenüber der Ringsitzfläche 10 und gegenüber der ringförmigen Auflage 12 stellt dabei sicher, daß eine leckfreie Abdichtung des Zwei-Wege-Ventils sowohl nach innen als auch nach außen gegeben ist. Darüber hinaus ist die Vorspannung gegenüber der ringförmigen Auflage 12 auch deshalb vorteilhaft, weil so die Möglichkeit besteht einen Vordruck für die austragbaren Flüssigkeiten festzulegen. Es ist zum Überwinden der Vorspannung gegenüber der ringförmigen Auflage 12 erforderlich, daß auf die auszutragende Flüssigkeit unter einem entsprechend höheren Druck steht. Je höher die Vorspannung dabei ist, desto größer ist der Vordruck, der erforderlich ist bevor ein Austrag stattfindet. Dadurch wird insbesondere bei Verwendung des Ventils in Kombination mit einer Sprühdüse erreicht, daß ein ausreichender Sprühdruck in der Flüssigkeit enthalten ist um eine gleichmässige und gute Zerstäubung zu erhalten.

Die Membranscheibe 4 ist vorteilhafterweise aus einem elastischen Material hergestellt. Es können hier sowohl Naturgummi, Silikongummi, synthetische Elastomere als auch thermoplastische Elastomere Verwendung finden. Insbesondere die Verwendung von thermoplastischen Elastomeren ist vorteilhaft, da diese ebenso verarbeitet werden können wie thermoplastische Polymere (z.B. Spritzguß) aber dennoch elastomere Eigenschaften aufweisen die zumindest den elastomeren Eigenschaften von Naturgumm, synthetischen Elastomeren oder Silikongummi gleichwertig sind. Besonders vorteilhaft ist es, wenn ein sogenanntes Zwei-Komponenten-Spritzgießverfahren zur Herstellung der Membranscheibe 4 verwendet wird. Es kann dann eine Membranscheibe 4 hergestellt werden, die aus zwei Ringen besteht, die eine unterschiedliche Federhärte aufweisen. Es kann beispielsweise der Außenring eine härtere Federeigenschaft aufweisen als der Innenring, der weicher ausgebildet sein kann. So könnte beispielsweise der Außenring eine Shorehärte von ca. Shore A90 und der Innenring eine Schorhärte von ca. Shore A35 aufweisen. Dabei sind die vorstehend gemachten Angaben betreffend der Shorehärte beispielhaft genannte Werte. Die Shorehärte des einzelnen Rings muß an die Verhältnisse des einzelnen Ventils und die gewünschten Vorspannkräfte gegenüber der ringförmigen Auflage 12 und der Ringsitzfläche 10 passend gewählt werden. Dennoch ermöglicht es die geeignete Auswahl der Härte des entsprechenden Ringes den Ansaug- und den Pressdruck beim Austragshub so zu regeln, daß die Zerstäuber- oder Spenderfunktion optimiert werden kann.

Bei vielen Anwendungen kann es von Vorteil sein, wenn die Leckage-Dichtheit des Zwei-Wege-Ventils auch bei Unterdruck gewährleistet ist. Bei dem vorstehend beschriebenen Ventil kann eine Leckage-Dichtheit bis zu Drücken von ca. 300 mbar hinunter gewährleistet werden, wenn auf den Kanalabschnitt 6b verzichtet wird. Wenn aufgrund eines geringen Außendrucks - beispielsweise aufgrund der Verwendung des Ventils bzw. des Flüssigkeitsspenders im Innenraum eines Flugzeuges - äußerlich ein Unterdruck herrscht, so herrscht aufgrund der fluidischen Verbindung des Anschlußkanals 14 mit dem dritten Anschluß 3 auch dort ein Unterdruck. Der Teil der Membranscheibe 4, der an der ventilgehäuseseiten, ringförmigen Auflage 12 anliegt, wird aufgrund der Druckdifferenz zwischen dem zweiten Ringkanal 13 und dem verringerten Druck im Anschlußkanal 14 stärker angedrückt. Im Zentrum 4b herrscht aber derselbe höhere Druck wie im zweiten Ringkanal 13. Da jedoch die Oberfläche des Ringkanales 13 mit dem höheren Druck größer ist als der erste Ringkanal 11 ist die Druckkraft, die im nach innen schließenden Sinne wirkt, ebenfalls größer als die Gegenkraft gegenüber dem Druck im Ringkanal 13. Aufgrund dieser Tatsache ist das Zwei-Wege-Ventil auch dann noch dichtend, wenn es gegenüber einem außen anliegenden Unterdruck arbeiten und abdichten soll.

Die Funktionsweise des Zwei-Wege-Ventils wird nachstehend beschrieben:

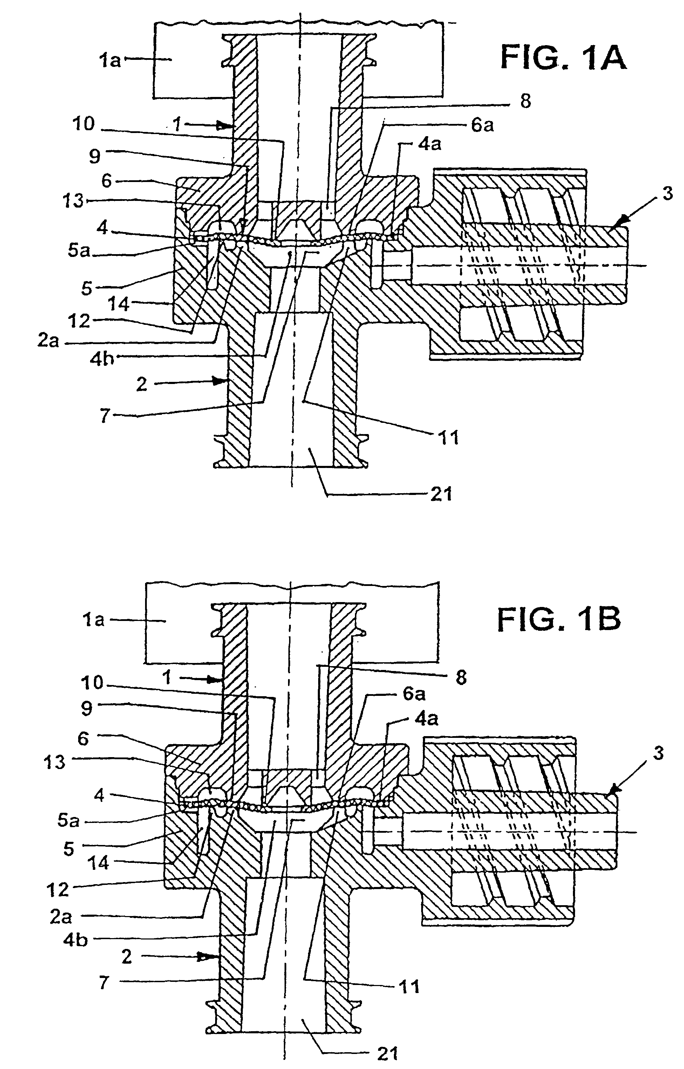

Während eines Saughubes des Kolbens im Zylinderraum 21 nach unten (Fig. 1A), strömt Flüssigkeit durch den ersten Anschluß 1 und durch die im Zentrum mittig offene Membranscheibe 4 in den Zylinderraum 21. Der Strömungsspalt an der Ringsitzfläche 10 entsteht durch die Saugkraft, die im Zentrum 4b und in der hohlen Ausnehmung 19 aufgebaut wird.

Nach Umkehr der Kolbenbewegung in die Richtung senkrecht nach oben (Fig. 1B) drückt die Flüssigkeit durch das Zentrum 4b auf die größere Kreisringfläche der ringförmigen Auflage 12, die zwischen der zusätzlichen Einspannung 9 und dem radialen Ringrand 4a besteht.

In Anbetracht der Vorspannung, mit der die Membranscheibe 4 auf der deckelseitigen, ringförmigen Auflage 12 aufliegt, genügt der dort herrschende Druck, um eine Öffnung der Membranscheibe 4 zu erzielen, wobei der Druck sich bis in den Anschlußkanal 14 fortpflanzt.

Beim Ansaugen der Flüssigkeit schließt die Membranscheibe 4 wegen des großen Durchmessers an der deckelseitigen, ringförmigen Auflage 12.

## Patentansprüche

1. Zwei-Wege-Ventil, insbesondere für Flüssigkeitsspender, mit einem ersten Anschluß (1) für einen Flüssigkeitsvorratsbehälter (1a), einem zweiten Anschluß (2) für eine Pumpe zum Saugen oder Drücken und mit einem dritten Anschluß (3) für einen Flüssigkeitsverbraucher, wobei eine Membranscheibe (4) an ihrem radial äußeren Ringrand (4a) zwischen einem Ventilgehäuse (5) und einem Deckelgehäuse (6) eingespannt ist und flach gegen eine Ringsitzfläche (10) anliegt, in deren Bereich sich eine axiale Öffnung (8) für die Flüssigkeit befindet und wobei die Membranscheibe (4) deckelseitig gegen eine Gegen-Ringsitzfläche (6a) anliegt, wobei zwischen dem radial äußeren Ringrand (4a) und der im Durchmesser radial kleineren Ringsitzfläche (10) eine zusätzliche beidseitige Einspannung (9) der im Zentrum (4b) offenen Membranscheibe (4), bestehend aus der Gegen-Ringsitzfläche (6a) und einer weiteren Ringsitzfläche (2a), vorgesehen ist und wobei neben der zusätzlichen Einspannung (9) ein erster Ringkanal (11) gebildet ist, der in den dritten Anschluß (3) mündet.

2. Zwei-Wege-Ventil nach Anspruch 1, **dadurch gekennzeichnet, daß** zwischen dem radial äußeren Ringrand (4a) der Membranscheibe (4) und der zusätzlichen, radial inneren Einspannung (9) die Membranscheibe (4) gegen eine einseitige, ventilgehäuseseitige, ringförmige Auflage (12) anliegt.

3. Zwei-Wege-Ventil nach Anspruch 2, **dadurch gekennzeichnet, daß** der ringförmigen Auflage (12) am Ventilgehäuse (5) ein Ringkanal (13) des Deckelgehäuses (6) gegenüberliegt.

4. Zwei-Wege-Ventil nach Anspruch 3, **dadurch gekennzeichnet, daß** innerhalb des Ventilgehäuses (5), an die Auflage (12) radial angrenzend, ein dem Ringkanal (13) des Deckelgehäuses (6) gegenüberliegender Anschlußkanal (14) vorgesehen ist.

5. Zwei-Wege-Ventil nach Anspruch 4, **dadurch gekennzeichnet, daß** auf der Seite des Ventilgehäuses (5) der ringförmige Anschlußkanal (14) mit einer gegenüber der radialen Breite (15) größeren axialen Länge (16) vorgesehen ist.

6. Zwei-Wege-Ventil nach einem der Ansprüche 4 bis 5, **dadurch gekennzeichnet, daß** der Ringkanal (13) an einer Umfangsstelle (17) durch einen den Durchmesser der Membranscheibe (4) umgebenden Kanalabschnitt (6b) im Deckelgehäuse (6) und einen solchen Kanalabschnitt (5a) im Ventilgehäuse (5) mit dem Anschlußkanal (14) verbunden ist.

7. Zwei-Wege-Ventil nach einem der Ansprüche 1 bis 6, **dadurch gekennzeichnet, daß** in dem Deckelgehäuse (6) die Ringsitzfläche (10) mittels einer nach innen hohlen Ausnehmung (18) eines Kernteils (19) des Deckelgehäuses (6) gebildet ist.

8. Zwei-Wege-Ventil nach Anspruch 7, **dadurch gekennzeichnet, daß** um den Kernteil (19) des Deckelgehäuses (6) einzelne ringsegmentförmige Ausnehmungen oder mehrere zylindrische Löcher (20) angeordnet sind, die den ersten Anschluß (1) mit dem Zentrum (4b) bei geöffnetem Ventil zum Ansaugen von Flüssigkeit verbinden.

## Claims

1. Two-way valve, particularly for liquid dispensers, with a first connection (1) for a liquid reservoir (1a), a second connection (2) for a pump for suction or compression and a third connection (3) for a liquid consuming means, a diaphragm disk (4) being clamped on its radially outer annular edge (4a) between a valve housing (5) and a cover housing (6) and engaging flat against an annular seat (10), in whose vicinity is located an axial opening (8) for the liquid and where the diaphragm disk (4) engages on the cover side against a mating annular seat (6a), wherein between the radially outer annular edge (4a) and the radially smaller diameter annular seat (10) is provided an additional, bilateral clamping (9) of the diaphragm disk (4) open in the centre (4b) and which comprises the mating annular seat (6a) and another annular seat (2a) and wherein besides the additional clamping (9) a first annular channel (11) is formed, which issues into the third connection (3).

2. Two-way valve according to claim 1, **characterized in that** between the radially outer annular edge (4a) of the diaphragm disk (4) and the additional, radially inner clamping (9), the diaphragm disk (4) engages against a unilateral, valve housing-side, annular bearing surface (12).

3. Two-way valve according to claim 2, **characterized in that** an annular channel (13) of the cover housing (6) faces the annular bearing surface (12) on the valve housing (5).

4. Two-way valve according to claim 3, **characterized in that**, radially adjacent to the bearing surface (12), within the valve housing (5) is provided a connection channel (14) facing the annular channel (13) of the cover housing (6).

5. Two-way valve according to claim 4, **characterized in that** on the side of the valve housing (5), the annular connection channel (14) is provided with an axial length (16) larger than the radial width (15).

6. Two-way valve according to one of the claims 4 to 5, **characterized in that** at a circumferential point (17), the annular channel (13) is connected by a channel section (6b) in the cover housing (6) surrounding the diameter of the diaphragm disk (4) and a similar channel section (5a) in the valve housing (5) to the connection channel (14).

7. Two-way valve according to one of the claims 1 to 6, **characterized in that** the annular seat (10) in the cover housing (6) is formed by means of an inwardly hollow recess (18) of a core part (19) of the cover housing (6).

8. Two-way valve according to claim 7, **characterized in that** individual ring segmental recesses or several cylindrical holes (20) are arranged around the core part (19) of the cover housing (6) and link the first connection (1) to the centre (4b) when the valve is open for liquid suction purposes.

## Revendications

1. Soupape à deux voies, notamment pour distributeurs de liquides, avec un premier raccord (1) pour un récipient-réservoir pour liquides (1a), un deuxième raccord (2) pour une pompe aspirante et refoulante et avec un troisième raccord (3) pour un moyen de consommation de liquide, un disque de membrane (4) étant tendu par son bord annulaire (4a) radialement extérieur entre un boîtier de soupape (5) et un boîtier de couvercle (6) et étant adjacent de manière plate à une surface de siège annulaire (10), dans le domaine de laquelle se trouve une ouverture (8) axiale pour le liquide, le disque de membrane (4) étant adjacent côté couvercle à une contre-surface de siège annulaire (6a), étant prévu entre le bord annulaire (4a) radialement extérieur et la surface de siège annulaire (10) radialement plus petite en diamètre un moyen de serrage (9) bilatéral additionnel du disque de membrane (4) ouvert au centre (4b), moyen qui est composé de la contre-surface de siège annulaire (6a) et d'une autre surface de siège annulaire (2a), et étant formé à côté du moyen de serrage (9) additionnel un premier conduit annulaire (11), qui débouche dans le troisième raccord (3).

2. Soupape à deux voies d'après la revendication 1, **caractérisé en ce que** entre le bord annulaire (4a) radialement extérieur du disque de membrane (4) et le moyen de serrage (9) additionnel radialement intérieur le disque de membrane (4) est adjacent à un appui (12) annulaire, unilatéral, du côté du boîtier de soupape.

3. Soupape à deux voies d'après la revendication 2, **caractérisé en ce que**, en face de l'appui (12) annulaire sur le boîtier de soupape (5) se trouve un conduit annulaire (13) du boîtier de couvercle (6).

4. Soupape à deux voies d'après la revendication 3, **caractérisé en ce qu'**à l'intérieur du boîtier de soupape (5), radialement à proximité immédiate de l'appui (12) est prévu un conduit de raccordement (14) situé en face du conduit annulaire (13) du boîtier de couvercle (6).

5. Soupape à deux voies d'après la revendication 4, **caractérisé en ce que** du côté du boîtier de soupape (5) est prévu le conduit de raccordement (14) annulaire présentant une longueur (16) axiale supérieure à la largeur (15) radiale.

6. Soupape à deux voies d'après une des revendications de 4 à 5, **caractérisé en ce que** le conduit annulaire (13) est raccordé dans le domaine d'une zone périphérique (17) au conduit de raccordement (14) au moyen d'une section de conduit (6b) dans le boîtier de couvercle (6), section entourant le diamètre du disque de membrane (4) et au moyen d'une section de conduit (5a) analogue dans le boîtier de soupape (5).

7. Soupape à deux voies d'après une des revendications de 1 à 6, **caractérisé en ce que** dans le boîtier de couvercle (6) la surface de siège annulaire (10) est formée au moyen d'un creux (18) vide vers l'intérieur, d'un élément de noyau (19) du boîtier de couvercle (6).

8. Soupape à deux voies d'après la revendication 7, **caractérisé en ce qu'**autour de l'élément de noyau (19) du boîtier de couvercle (6) sont disposés des creux individuels en forme de segments d'anneau ou bien plusieurs forures (20) cylindriques, qui relient pour une aspiration de liquide le premier raccord (1) avec le centre (4b), la soupape étant ouverte.
